# FASCICULE DE BREVET EUROPEEN

(11) **EP 3 982 902 B1**
(45) Date de publication et mention de la délivrance du brevet: **28.08.2024**
(21) Numéro de dépôt: 19745675.9
(22) Date de dépôt: 11.06.2019
(51) Int. Cl.: A61H 9/00

(54) **DISPOSITIF D'ASSISTANCE CIRCULATOIRE PULSATILE NON INVASIF**
NICHT-INVASIVES, PULSIERENDES GERÄT FÜR KREISLAUFUNTERSTÜTZUNG
NON-INVASIVE PULSATILE DEVICE FOR CIRCULATORY ASSISTANCE

(43) Date de publication de la demande: 20.04.2022
(73) Titulaire: Cardio Innovative Systems, 75016 Paris (FR)
(72) Inventeur: LE BLE, Renan, 27430 Saint-Pierre-du-Vauvray (FR); DIXMIER, Michel, 75018 Paris (FR); CHASTANIER, Pierre, 75008 Paris (FR); BAILLIART, Olivier, 75016 Paris (FR)
(74) Mandataire: Gauchet, Fabien Roland
(86) Numéro de dépôt international: PCT/FR2019/051401
(87) Numéro de publication internationale: WO 2020/249875

(56) Documents cités:
- WO-A1-2014/068288
- WO-A2-2007/078845
- KR-U- 20110 002 704
- SU-A1- 1 250 293
- US-A- 5 891 065
- US-B1- 8 465 444

## Description

### DOMAINE TECHNIQUE DE L'INVENTION

L'invention concerne un dispositif d'assistance circulatoire pulsatile non invasif.

### ETAT DE LA TECHNIQUE ANTERIEURE

Le système circulatoire constitue un circuit hydraulique, fermé, sous pression, tapissé intérieurement par des cellules endothéliales. Cet endothélium est continuellement et de façon pulsatile, soumis à des forces tangentielles de cisaillement indispensables au maintien de sa fonction physiologique : tonus vasculaire grâce à la synthèse de monoxyde d'azote, coagulation du sang, réponse inflammatoire, lutte contre l'athérosclérose, système immunitaire, angiogenèse et apoptose.

Toute altération pathologique de cette fonction endothéliale entraîne un dysfonctionnement du système aux conséquences parfois dramatiques.

Il existe des systèmes d'assistance cardiaque qui sont utilisés pour remplacer partiellement ou totalement l'activité cardiaque lors d'opération chirurgicale ou pour retrouver cette activité lorsque le coeur est arrêté ou trop faible. Ces systèmes d'assistance sont, pour la plupart, des systèmes invasifs : ils nécessitent soit l'introduction d'un outil dans le corps d'un sujet, cet outil étant ensuite utilisé pour créer des pulsations, soit le prélèvement du sang du sujet et le traitement du sang prélevé dans une machine volumineuse à l'extérieur du corps puis l'injection du sang dans le corps du sujet. Dans tous les cas, les systèmes actuels sont coûteux et compliqués à mettre en oeuvre puisqu'ils nécessitent l'intervention de spécialistes.

Un système non invasif a été décrit dans le document WO2010/070018 ainsi que dans le document WO2014/080016. Ces documents décrivent un dispositif d'assistance circulatoire pulsatile non invasif comportant une structure composée d'une ou plusieurs poches reliées à une console permettant de générer des ondes pulsatiles au niveau de la ou des poches de sorte à favoriser une circulation d'un volume sanguin dans au moins une partie d'un corps d'un sujet.

Le document US5891065 A divulgue un dispositif selon le préambule de la revendication 1.

Dans le cas d'un dispositif comportant une seule poche, l'inconvénient est l'impossibilité de contrôler avec précision la répartition de l'onde pulsatile ou de la moduler en fonction des zones d'appui de la poche sur la partie du corps du sujet à traiter.

Dans le cas d'un dispositif comportant plusieurs poches, les poches sont positionnées de manière adjacente deux à deux, ce qui permet de résoudre partiellement l'inconvénient précédent de la poche unique. Toutefois, il y a une perte de la continuité dans la propagation des ondes de pulsation générées au niveau de la jonction entre deux poches adjacentes. De plus, cette jonction entraine, lors du fonctionnement du dispositif, un effet garrot préjudiciable au sujet.

D'autres systèmes non invasifs dits ECP (pour External Counter Pulsation selon la terminologie anglo-saxonne signifiant « Contre-pulsion externe ») réalisent une contre-pulsion externe induisant un reflux sanguin. De façon connue ces systèmes agissent comme un garrot sur la partie inférieure du corps, ce qui dirige le sang en amont, vers les organes vitaux, dont le coeur. En d'autres termes, l'ECP interrompt la circulation sanguine par effet garrot, avec une contre-pression de l'ordre d'au moins 173,3 millibars (130 mmHg).

Dans certains systèmes ECP, l'effet garrot est devenu séquentiel, puis il a été synchronisé sur la fréquence cardiaque, l'effet garrot étant alors provoqué durant la diastole. C'est un effet de type mécanique des fluides qui est ici opéré : création d'une onde de pression rétrograde aortique qui augmente la perfusion coronaire ; effet garrot, sans augmentation significative du retour veineux, donc sans effet sur la pré-charge ventriculaire droite et sur le débit cardiaque. Cette technique nécessite une pression, appliquée sur la partie inférieure du corps, de l'ordre de 266,6 à 399,9 millibars (200 à 300 mmHg), ce qui est largement supérieur à la pression systolique physiologique du sujet. Cette pression avec effet de garrot séquentiel induit une compression des vaisseaux, ce qui provoque de facto un effet de pression mécanique sur les vaisseaux. Un effet délétère sur les cellules endothéliales et les vaisseaux sanguins est malheureusement rencontré par cette technique.

Il est aussi connu des dispositifs et procédés de presso-thérapie qui consistent généralement en une méthode de drainage veino-lymphatique. De façon usuelle, le drainage veino-lymphatique par une méthode de presso-thérapie utilise des appareils à gradient de pression physiologique, avec des bottes gonflables activées par un compresseur à air (la pression augmente progressivement, allant de 40 à 80 millibars). Le but de ce massage est d'activer la circulation de la lymphe, de stimuler le système de défense immunitaire, et d'entraîner une détente profonde. Ainsi le phénomène de jambes lourdes et de cellulite s'amenuise petit à petit. Toutefois, la presso-thérapie a pour inconvénient de comprimer sur une longue durée les vaisseaux sanguins ce qui entrave la circulation du sang, en dehors de tout rythme cardiaque. D'autre part, de sorte à activer la circulation de la lymphe, la presso-thérapie nécessite de comprimer de manière importante le réseau lymphatique.

### EXPOSE DE L'INVENTION

Un but de l'invention est de fournir un dispositif d'assistance circulatoire pulsatile non invasif qui permet la propagation homogène, continue et régulière des ondes de pulsation tout en permettant un contrôle précis et/ou une modulation des ondes de pulsation en fonction des zones d'appui du dispositif sur la partie du corps du sujet.

La présente invention propose un dispositif d'assistance circulatoire pulsatile non invasif destiné à favoriser une circulation d'un volume sanguin dans au moins une partie d'un corps d'un sujet selon la revendication 1.

Avantageusement, mais facultativement, le dispositif selon l'invention présente au moins l'une des caractéristiques techniques suivantes :
- les poches de la série de poches sont montées en écailles ;
- chacune des poches comprend une couche interne souple élastique du côté du corps du patient et une couche externe plus rigide ;
- chacune des poches comporte un conduit d'admission d'un fluide de pulsation en connexion fluidique avec les moyens de génération d'une pulsation ;
- chacun des coussins gonflables comporte un conduit d'admission d'un fluide de pulsation en connexion fluidique avec les moyens de génération d'une pulsation ;
- l'enveloppe multicouche comporte une couche externe souple non extensible ;
- le dispositif comporte un masque agencé de sorte à être disposé sur au moins une partie d'un visage d'un sujet ;
- le dispositif comporte un pantalon ;
- le dispositif comporte une veste ;
- le dispositif comporte un gant ; et,
- le dispositif comporte une botte ou une chaussette.

Il est aussi prévu, selon l'invention, un ensemble d'assistance circulatoire pulsatile non invasif couvrant plusieurs parties corps d'un sujet comprenant au moins un dispositif présentant au moins l'une des caractéristiques techniques précédentes pour chacune des parties du corps du sujet.

### BREVE DESCRIPTION DES FIGURES

D'autres caractéristiques et avantages de l'invention ressortiront à la lecture de la description qui suit d'un mode de réalisation de l'invention. Aux dessins annexés :
- la figure 1 est une représentation schématique d'un dispositif d'assistance circulatoire pulsatile non invasif non conforme à l'invention, mais à des fins d'illustration ; ;
- la figure 2 est une représentation schématique d'un exemple de structure multicouche formant une poche mise en oeuvre dans le dispositif de la figure 1 ;
- la figure 3 est une représentation schématique d'un masque pulsatile selon l'invention ;
- la figure 4 est une représentation schématique d'un pantalon pulsatile selon l'invention ; et,
- la figure 5 est une représentation schématique d'un mode de réalisation du dispositif d'assistance circulatoire pulsatile non invasif selon l'invention.

Pour plus de clarté, les éléments identiques ou similaires sont repérés par des signes de référence identiques sur l'ensemble des figures.

### DESCRIPTION DETAILLEE D'UN MODE DE REALISATION

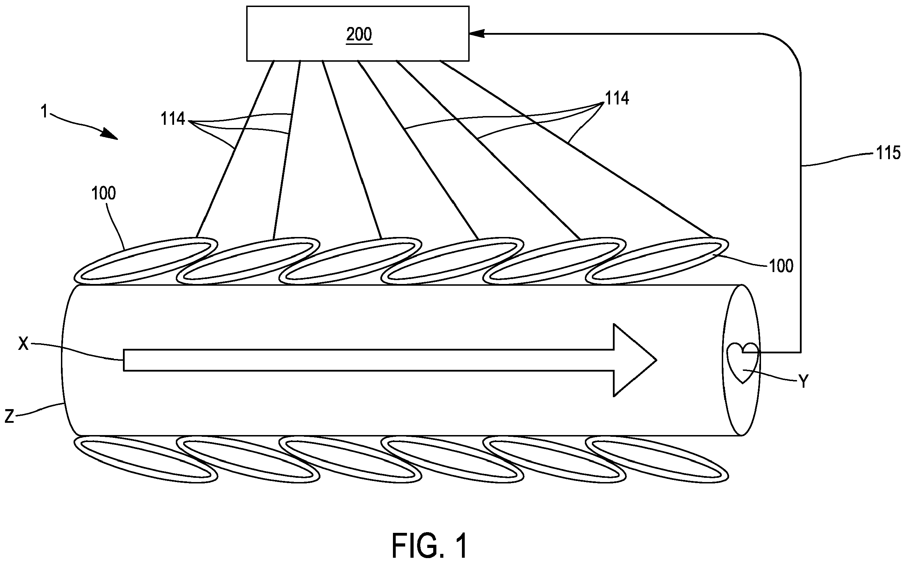

En référence à la figure 1, nous allons décrire un dispositif d'assistance circulatoire pulsatile non invasif 1 (non conforme à l'invention). Le dispositif d'assistance circulatoire pulsatile non invasif 1 est destiné à être positionné sur une partie Z d'un corps d'un sujet de sorte à favoriser, lors d'une mise en oeuvre, une circulation d'un volume sanguin dans la partie Z du corps du sujet, dans la direction X vers le coeur Y du sujet.

La partie Z du corps du sujet est schématisée ici par une portion de tube cylindrique de révolution qui est ici utilisée à titre purement illustratif. En particulier, la partie Z peut être un membre supérieur ou inférieur du sujet, ou encore le tronc ou la tête de ce dernier. La structure du dispositif d'assistance circulatoire pulsatile non invasif 1 permet à ce que ce dernier puisse se conformer à toutes formes et géométries de la partie Z du corps du sujet à équiper d'un tel dispositif d'assistance circulatoire pulsatile non invasif 1.

Le dispositif d'assistance circulatoire pulsatile non invasif 1 comporte ici une série de poches 100 adjacentes deux à deux. La série de poches 100 illustrée à la figure 1 comprend six poches 100 uniformément réparties le long de la partie Z du corps du sujet. Le nombre de poches 100 varie en fonction de la partie Z du corps du sujet qui est visée, il peut être compris entre un et six, ou bien être supérieur à six. Chacune des poches 100 entoure, ici, la partie Z du corps du sujet. Chacune des poches 100 comportent un conduit d'admission 114 qui permet de connecter fluidiquement la poche 100 à une console pulsatile 200 formant moyens de génération d'une pulsation. La même console pulsatile 200 du dispositif d'assistance circulatoire pulsatile non invasif 1 contrôle l'ensemble des poches 100 de la série de poches 100, les différentes poches 100 étant indépendantes les unes des autres. Les moyens de génération d'une pulsation 200 formant la console pulsatile peuvent avoir différentes structures qui sont décrites dans le document WO 2010/070018 auquel il est possible de se référer pour de plus amples informations. Ils ne seront donc pas décrits plus avant ici.

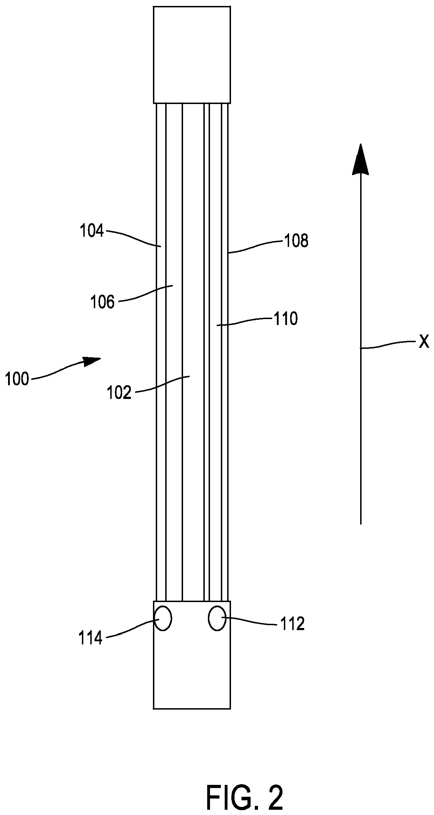

Les poches 100 sont donc gonflables via leur conduit d'admission 114. Pour cela, elles présentent une paroi élastiquement déformable dans un mode de réalisation le plus simple. En référence à la figure 2, nous décrirons ultérieurement un autre mode de réalisation des poches 100.

**Sur** la partie Z du corps du sujet, les poches 100 adjacentes deux à deux de la série de poches 100 sont montées de telle sorte que l'une des poches adjacentes deux à deux recouvre au moins partiellement l'autre des poches adjacentes deux à deux. Ce recouvrement partiel permet d'obtenir une continuité dans la propagation des ondes pulsatiles générées par la console pulsatile 200. En particulier, le dispositif d'assistance circulatoire pulsatile non invasif 1 ainsi réalisé permet, lors d'un fonctionnement, une action massante centripète (selon la direction X) en vagues, sans interruption, totale, régulière et homogène, tout en gardant la possibilité d'exercer des pressions différenciées, voire nulles si nécessaire, sur certaines zones de la partie Z du corps du sujet, en fonction des besoins (présence de brûlure, plaie, oedème, etc...). De préférence, la console pulsatile 200 est agencée de sorte à synchroniser les ondes pulsatiles qu'elle génère avec un signal physiologique 115 qu'elle reçoit à partir d'un capteur placé sur le sujet. Le signal physiologique provient par exemple du rythme cardiaque et en particulier correspond à la diastole dudit rythme cardiaque. De plus, une information de la pression artérielle du sujet peut être relevée en parallèle de sorte à adapter les pressions générées par la console 200 audit sujet. Dans le cas d'une arythmie cardiaque, le signal physiologique peut provenir du cycle respiratoire du sujet.

Dans un agencement particulier, les poches 100 sont montées en écailles comme illustré à la figure 1. D'autres agencements des poches 100 sont envisageables pour le dispositif d'assistance circulatoire pulsatile non invasif 1, tant que l'une des poches adjacentes deux à deux recouvre au moins partiellement l'autre des poches adjacentes deux à deux dans la série de poches 100 formant le dispositif d'assistance circulatoire pulsatile non invasif 1.

La figure 2 est une représentation schématique d'un mode de réalisation d'une structure multicouche formant chacune des poches 100 mise en oeuvre dans le dispositif d'assistance circulatoire pulsatile non invasif 1.

La structure multicouche formant chacune des poches 100 représentée en figure 1 comporte :
- une couche interne 102 réalisée en un matériau élastique, par exemple en néoprène, polyuréthane, latex ...
- une couche externe 104 formée d'un coussin gonflable pouvant présenter une paroi externe constituée d'un matériau rigide ou souple non extensible dans son plan guidant la propagation des ondes de compression vers l'intérieur du corps, et
- une couche intermédiaire 106 contenant un fluide, qui peut être aussi un gel, des micro billes ou la combinaison des deux permettant la propagation d'une vague de pression pulsatile progressive et vers le coeur selon la direction naturelle et physiologique du drainage veineux et lymphatique dans la partie sur laquelle est appliquée ladite structure. Il est considéré dans la suite de la description que la direction naturelle et physiologique du drainage veineux et lymphatique est la direction X représentée sur les figures 1 et 2.

La structure multicouche formant chacune des poches 100 comporte en outre une couche additionnelle 108, comportant une cavité 110 en matériel biocompatible, et comportant une paroi microporeuse destinée à venir en contact du corps du sujet. La cavité 110 peut être remplie d'un produit fluide biocompatible et/ou biologique à travers un connecteur 112. La paroi microporeuse est en contact direct avec la peau du corps du sujet. Lors des pulsations, le produit contenu dans l'espace 110 de cette couche 108 est appliqué sur le corps du sujet par passage au travers de la partie microporeuse.

La couche externe 104 est en connexion fluidique de manière étanche avec les moyens de pulsation 200 pour créer des ondes de pulsation dans les poches 100 du dispositif d'assistance circulatoire pulsatile non invasif 1 selon l'invention grâce à un conduit d'admission 114.

Pour assurer la propagation des pulsations le long de la partie du corps sur laquelle la poche 100 est appliquée, la couche intermédiaire 106 comporte un produit de consistance variable, gélatineux, granuleux ou autre, et distribuant chacune des pulsations progressivement le long de ladite structure multicouche formant chacune des poches 100 dans la direction X.

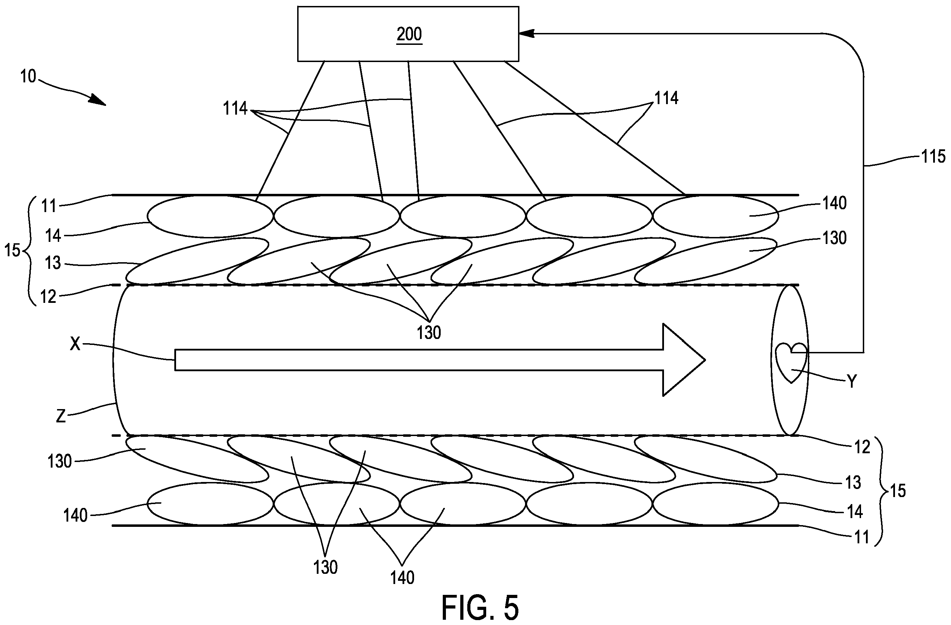

En relation avec la figure 5, nous allons maintenant décrire un mode de réalisation d'un dispositif d'assistance circulatoire pulsatile non invasif 10 selon l'invention.

Le dispositif d'assistance circulatoire pulsatile non invasif 10 selon l'invention comporte une enveloppe 15 multicouche qui vient entourer la partie Z du corps du sujet lors d'une mise en place du dispositif d'assistance circulatoire pulsatile non invasif 10 selon l'invention. L'enveloppe 15 comporte :
- Une couche interne 12 qui est formée en un matériau élastique, par exemple en néoprène, polyuréthane, latex ...;
- Une couche de répartition 13 comportant ici une série de poches 130 adjacentes deux à deux ;
- Une couche de génération 14 comportant ici une série de coussins 140 gonflables adjacents deux à deux; et,
- Une couche externe 11 en un matériau souple non extensible dans son plan ou en un matériau rigide.

La couche interne 12 se présente sous la forme générale d'un film destiné à venir en contact avec la partie Z du corps du sujet et pouvant se déformer et s'étendre lors d'une utilisation du dispositif d'assistance circulatoire pulsatile non invasif 10 selon l'invention. Selon une variante de réalisation, la couche interne 12 est microporeuse et peut être imprégnée d'un produit fluide biocompatible et/ou biologique. Lors des pulsations, le produit imprégnant les micropores de la couche interne 12 est appliqué sur le corps du sujet.

La série de poches 130, formant la couche de répartition 13 et illustrée à la figure 5, comprend, ici, six poches 130 uniformément réparties le long de la partie Z du corps du sujet. Chacune des poches 130 entoure, ici, la partie Z du corps du sujet et est séparée de ladite partie Z du corps du sujet par la couche interne 12 servant alors d'interface entre chacune des poches 130 et la partie Z du corps du sujet, les différentes poches 130 étant indépendantes les unes des autres. Les poches 130 présentent une paroi élastiquement déformable et sont remplies avec un fluide incompressible permettant la propagation d'une vague de pression pulsatile progressive et vers le coeur selon la direction naturelle et physiologique du drainage veineux et lymphatique dans la partie Z sur laquelle est appliqué le dispositif d'assistance circulatoire pulsatile non invasif 10 selon l'invention. Par exemple, ce fluide incompressible est un fluide gélatineux, granuleux ou autre. Comme pour le premier exemple illustré en figure 1 et décrit précédemment, les poches 130 adjacentes deux à deux de la série de poches 130 sont montées de telle sorte que l'une des poches adjacentes deux à deux recouvre au moins partiellement l'autre des poches adjacentes deux à deux. Ce recouvrement partiel permet d'obtenir une continuité dans la propagation des ondes pulsatiles générées par la console pulsatile 200, tout en gardant la possibilité d'exercer des pressions différenciées, voire nulles si nécessaire, sur certaines zones de la partie Z du corps du sujet, en fonction des besoins (présence de brûlure, plaie, oedème, etc...).

La série de coussins 140, formant la couche de génération 14 et illustrée à la figure 5, comporte, ici six coussins 140 uniformément répartis le long de la partie Z du corps du sujet. Chacun des coussins 140 entoure, ici la couche de répartition 13 de sorte que cette dernière soit prise en sandwich entre la couche interne 12 et ladite couche de génération 14. Ainsi, la couche de génération 14 recouvre la couche de répartition 13. De plus, chacun des coussins 140 comporte un conduit d'admission 114 qui permet de connecter fluidiquement le coussin 140 à la console pulsatile 200 formant moyens de génération d'une pulsation. La même console pulsatile 200 du dispositif d'assistance circulatoire pulsatile non invasif 10 selon l'invention contrôle l'ensemble des coussins 140 de la série de coussins 140, les différents coussins 140 étant indépendants les uns des autres. Les coussins 140 sont donc gonflables via leur conduit d'admission 114. Pour cela, ils présentent une paroi élastiquement déformable. Le fluide utilisé dans les coussins 140 est de l'air, tout autre fluide étant utilisable en variante. Ainsi, le dispositif d'assistance circulatoire pulsatile non invasif 10 selon l'invention ainsi réalisé permet lui aussi, lors d'un fonctionnement, une action massante centripète (selon la direction X) en vagues, sans interruption, totale, régulière et homogène, tout en gardant la possibilité d'exercer des pressions différenciées, voire nulles si nécessaire, sur certaines zones de la partie Z du corps du sujet, en fonction des besoins (présence de brûlure, plaie, oedème, etc...). Les pressions sont alors transmises depuis la couche de génération 14 vers la partie Z du corps du sujet par la couche interne 12.

La couche externe 11 se présente sous la forme d'un film souple, comme pour la couche interne 12. La couche externe 11 n'est pas extensible dans son plan de sorte que les déformations de la série de coussins 140 gonflables générées par la console 200 au sein de la couche de génération 14 ne peuvent se faire au principal qu'en direction de la partie Z du corps du sujet, de sorte à produire les ondes de pression qui y seront appliquées via la couche de répartition 13. Toutefois, la couche externe 11 est suffisamment souple afin de permettre la mise en place du dispositif d'assistance circulatoire pulsatile non invasif 10 selon l'invention autour de la partie Z du corps du sujet.

Dans une variante de réalisation, le dispositif d'assistance circulatoire pulsatile non invasif 10 selon l'invention comporte des moyens de régulation d'une température au sein de l'enveloppe 15 multicouche, et en particulier au voisinage de la couche interne 12.

Nous allons maintenant décrire différents éléments pulsatiles selon l'invention.

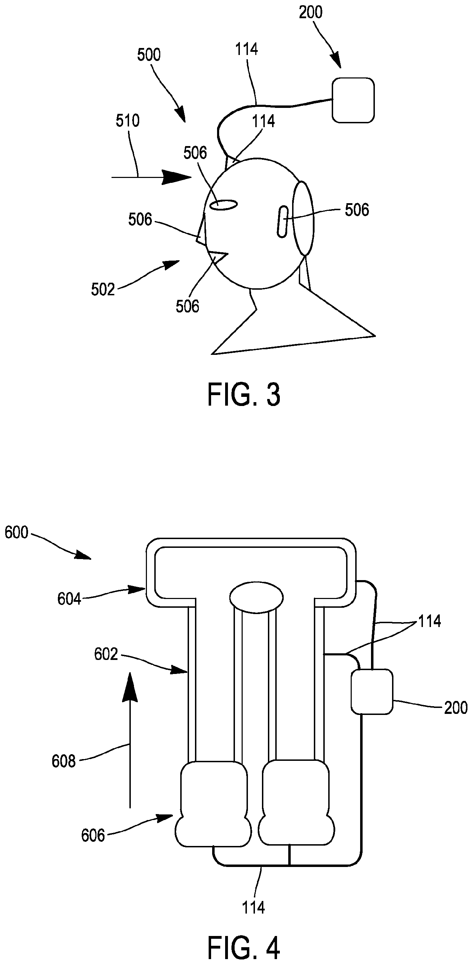

La figure 3 est une représentation schématique d'un masque pulsatile 500 selon l'invention. Le masque pulsatile 500 se compose d'une partie faciale 502 réalisée avec un dispositif d'assistance circulatoire pulsatile non invasif tel que décrit précédemment en référence aux figures 1, 2 et 5.

La partie faciale 502 peut posséder des ouvertures 506, aux niveaux orbital, buccal, nasal et auriculaire. Un ensemble de conduits d'admission 114 relie la console pulsatile 200 à l'ensemble des poches 100 de la partie faciale 502. Chacune des pulsations réalisées et pilotées à travers l'ensemble des poches 100 se propage progressivement dans la partie faciale 502 de sorte à réaliser un massage facial. Un axe horizontal matérialisé par la flèche 510 représente le trajet des ondes pulsatiles vers le circuit caverneux.

Le masque 500 sert d'assistance circulatoire pulsatile non-invasive pour traiter la stase veino-lymphatique du visage et du cou. Elle se porte appliquée au visage et en partie au cuir chevelu. La partie faciale 502 peut fonctionner en synchronisation rythmique et régulière et en harmonie avec les rythmes cardiorespiratoires du sujet.

Le masque 500 présente en outre les fonctions suivantes :
- En principale : la restauration et la réparation des effets secondaires du dysfonctionnement endothélial par application des forces de cisaillement synchronisées avec la diastole, réduisant les congestions lymphatique et veineuse ; et
- En secondaire : l'amélioration hémodynamique de la circulation sanguine.
- amélioration de la circulation cutanée, accélérant l'absorption et la pénétration de produits cosmétiques existants tels que des produits anti-âge ou soins de la peau.

La couche intérieure des poches 100 du masque 502 peut être modelée sur un masque biologique ou en matériau biocompatible, adaptée à la forme du visage et cou du sujet. La surface intérieure pourra être microporeuse permettant la diffusion vers la peau de fluides à caractère cosmétique avec ou sans variation de température des produits ou fluides utilisés, selon les indications.

La figure 4 est une représentation schématique d'un pantalon pulsatile 600 selon l'invention.

Le pantalon pulsatile 600 composé d'une partie jambes 602, d'une partie ceinture 604, et éventuellement d'une partie bottes 606. Chacune des trois parties précitées forme un dispositif d'assistance circulatoire pulsatile non invasif. Dans cette version, le pantalon 600 ne comporte pas de couche microporeuse.

Les ondes pulsatiles démarrent au niveau de la partie bottes 606 en provenance de la console pulsatile 200 via des ensembles de conduits d'admission 114 reliant fluidiquement les différentes poches 100 à ladite console pulsatile 200. Chacune des pulsations se propagent ensuite vers le coeur selon un axe matérialisé par la flèche 608.

Ce système, outre les fonctions précédemment décrites pour le masque 500 pulsatile, a une utilisation à la fois réparatrice et préventive :
- réparatrice du dysfonctionnement endothélial grâce aux forces de cisaillement en favorisant l'angiogenèse ; et
- préventive contre le dysfonctionnement endothélial.

Dans une version particulière, le pantalon pulsatile 600 peut comprendre une première couche en contact avec la peau à travers les vêtements personnels.

Des modifications de la partie dorsale de la ceinture 604 peuvent être envisagées pour assurer un massage du bas du rachis lombaire.

Les communications entre les différentes parties (pantalon, ceinture, jambes) sont coordonnées et synchronisées avec la diastole.

De la même manière peuvent être envisagés un manchon pulsatile, une veste pulsatile, des sous-vêtements pulsatiles, des bottes pulsatiles, des gants pulsatiles ainsi qu'une combinaison pulsatile complète formés à l'aide d'un ou plusieurs dispositifs d'assistance circulatoire pulsatile non invasifs.

Une combinaison pulsatile complète peut aussi être obtenue par assemblage d'un masque, d'une veste, d'un pantalon pulsatile, des gants pulsatiles et des chaussures pulsatiles. Dans ce cas, selon un premier mode de réalisation, chaque ensemble pulsatile peut être associé à des moyens de pulsation 200 dédiés. Selon un second mode de réalisation, des moyens de pulsation 200 uniques peuvent être utilisés pour tous les ensembles pulsatiles composant la combinaison pulsatile.

La propagation des impulsions pulsatiles est synchronisée à partir de plusieurs origines distales telles que les bottes pulsatiles ou des gants pulsatiles.

Chaque ensemble pulsatile peut être utilisé séparément en fonction des besoins du sujet.

Chaque dispositif pulsatile selon l'invention est un dispositif d'assistance circulatoire, non-invasif, permettant une réduction progressive de la capacitance veino-lymphatique stagnante. En augmentant la précharge du coeur droit par une augmentation du retour veino-lymphatique, le dispositif selon l'invention améliore la fonction cardiaque. En parallèle, par un effet de stimulation endothéliale et par un effet vasodilatateur, le dispositif selon l'invention, de par son utilisation, entraine une amélioration hémodynamique globale. À long terme les forces de cisaillement produites par le dispositif pulsatile selon l'invention vont restaurer et préserver la fonction endothéliale. Cette méthode plus physiologique, pouvant réduire la morbidité et la mortalité, est applicable chez l'enfant comme chez l'adulte ainsi que chez le sujet animal. En sus des forces de cisaillements produites, le dispositif pulsatile selon l'invention génère un effet drainant sur la partie Z du corps du sujet, ce qui améliore encore plus la microcirculation sanguine et donc la stimulation de l'endothélium veineux.

Bien entendu, il est possible d'apporter à l'invention de nombreuses modifications sans pour autant sortir du cadre de celle-ci.

## Revendications

1. Dispositif d'assistance circulatoire pulsatile non invasif (1;10;500;600) destiné à favoriser une circulation d'un volume sanguin dans au moins une partie (Z) d'un corps d'un sujet, le dispositif comprenant :
- une structure flexible (15), agencée de sorte à être appliquée sur au moins la partie du corps du sujet, comportant :
i. une couche de répartition (13) comprenant une première série de poches (130) adjacentes deux à deux s'étendant le long l'une de l'autre, l'une des poches adjacentes deux à deux recouvrant au moins partiellement l'autre des poches adjacentes deux à deux ;
ii. Une couche de génération (14), recouvrant la couche de répartition (130), comportant une deuxième série de poches gonflables (140) adjacentes deux à deux s'étendant le long l'une de l'autre ; et,
- des moyens de génération (200) d'une pulsation reliés fluidiquement à la structure flexible de manière étanche et agencés de sorte à créer des ondes de pulsation dans la deuxième série de poches de la couche de génération entre les couches interne et externe, **caractérisé en ce que** la première série de poches (130) de la couche de répartition comprenant des poches remplies avec un fluide incompressible permettant la propagation des ondes de pulsation créées dans la couche de génération par les moyens de génération (200).

2. Dispositif selon la revendication 1, **caractérisé en ce que** les poches de la deuxième série de poches sont montées en écailles.

3. Dispositif selon la revendication 1 ou 2, **caractérisé en ce que** chacune des poches de la deuxième série de poches (140) comprend une couche interne (102) souple élastique du côté du corps du patient et une couche externe (104) plus rigide.

4. Dispositif selon l'une des revendications 1 à 3, **caractérisé en ce que** chacun des coussins gonflables comporte un conduit d'admission (114) d'un fluide de pulsation en connexion fluidique avec les moyens de génération d'une pulsation.

5. Dispositif selon l'une des revendications 1 à 4, **caractérisé en ce que** la structure flexible comporte une couche externe (11) souple non extensible.

6. Dispositif selon l'une des revendications 1 à 5, **caractérisé en ce qu'**il comporte un masque (500) agencé de sorte à être disposé sur au moins une partie d'un visage d'un sujet.

7. Dispositif selon l'une des revendications 1 à 5, **caractérisé en ce qu'**il comporte un pantalon (600).

8. Dispositif selon l'une des revendications 1 à 5, **caractérisé en ce qu'**il comporte une veste.

9. Dispositif selon l'une des revendications 1 à 5, **caractérisé en ce qu'**il comporte un gant.

10. Dispositif selon l'une des revendications 1 à 5, **caractérisé en ce qu'**il comporte une botte (606) ou une chaussette.

11. Ensemble d'assistance circulatoire pulsatile non invasif couvrant plusieurs parties corps d'un sujet, **caractérisé en ce qu'**il comprend au moins un dispositif selon l'une des revendications 1 à 10 pour chacune des parties du corps du sujet.

## Patentansprüche

1. Nicht invasive, pulsierende Vorrichtung (1; 10; 500; 600) zur Unterstützung des Kreislaufs, die dazu bestimmt ist, die Zirkulation eines Blutvolumens in mindestens einem Teil (Z) des Körpers einer Person zu fördern, wobei die Vorrichtung Folgendes umfasst:
- eine flexible Struktur (15), die derart ausgebildet ist, dass sie auf mindestens einem Teil des Körpers der Person angebracht wird, und Folgendes umfasst:
i. eine Verteilungsschicht (13), die eine erste Reihe von paarweise aneinandergrenzenden Beuteln (130) umfasst, die sich längs zueinander erstrecken, wobei einer der paarweise aneinandergrenzenden Beutel den anderen der paarweise aneinandergrenzenden Beutel mindestens teilweise bedeckt;
ii. eine Erzeugungsschicht (14), die die Verteilungsschicht (130) bedeckt und eine zweite Reihe von paarweisen aneinandergrenzenden aufblasbaren Beuteln (140) umfasst, die sich längs zueinander erstrecken; und
- Erzeugungsmittel (200) für eine Pulsation, die fluidisch mit der flexiblen Struktur abdichtend verbunden und derart ausgebildet sind, dass sie Pulsationswellen in der zweiten Reihe von Beuteln der Erzeugungsschicht zwischen der inneren und der äußeren Schicht erzeugen, **dadurch gekennzeichnet, dass** die erste Reihe von Beuteln (130) der Verteilungsschicht Beutel umfasst, die mit einem nicht komprimierbaren Fluid gefüllt sind, das die Ausbreitung der Pulsationswellen ermöglicht, die durch die Erzeugungsmittel (200) in der Erzeugungsschicht erzeugt werden.

2. Vorrichtung nach Anspruch 1, **dadurch gekennzeichnet, dass** die Beutel der zweiten Reihe von Beuteln schuppenartig montiert sind.

3. Vorrichtung nach Anspruch 1 oder 2, **dadurch gekennzeichnet, dass** jeder der Beutel der zweiten Reihe von Beuteln (140) eine elastische, biegsame innere Schicht (102) auf der Seite des Körpers des Patienten und eine steifere äußere Schicht (104) umfasst.

4. Vorrichtung nach einem der Ansprüche 1 bis 3, **dadurch gekennzeichnet, dass** jedes der aufblasbaren Kissen einen Zugangskanal (114) für ein Pulsationsfluid aufweist, der in fluidischer Verbindung mit den Erzeugungsmitteln für eine Pulsation steht.

5. Vorrichtung nach einem der Ansprüche 1 bis 4, **dadurch gekennzeichnet, dass** die flexible Struktur eine nicht dehnbare, biegsame äußere Schicht (11) aufweist.

6. Vorrichtung nach einem der Ansprüche 1 bis 5, **dadurch gekennzeichnet, dass** sie eine Maske (500) aufweist, die derart ausgebildet ist, dass sie auf mindestens einem Teil des Gesichts einer Person angeordnet wird.

7. Vorrichtung nach einem der Ansprüche 1 bis 5, **dadurch gekennzeichnet, dass** sie eine Hose (600) aufweist.

8. Vorrichtung nach einem der Ansprüche 1 bis 5, **dadurch gekennzeichnet, dass** sie eine Jacke aufweist.

9. Vorrichtung nach einem der Ansprüche 1 bis 5, **dadurch gekennzeichnet, dass** sie einen Handschuh aufweist.

10. Vorrichtung nach einem der Ansprüche 1 bis 5, **dadurch gekennzeichnet, dass** sie einen Stiefel (606) oder einen Strumpf aufweist.

11. Nicht invasive, pulsierende Einheit zur Unterstützung des Kreislaufes, die mehrere Körperteile einer Person bedeckt, **dadurch gekennzeichnet, dass** sie mindestens eine Vorrichtung nach einem der Ansprüche 1 bis 10 für jeden der Körperteile der Person umfasst.

## Claims

1. Non-invasive pulsatile circulatory support device (1;10;500;600) intended to promote circulation of a blood volume in at least a part (Z) of a subject's body, the device comprising:
- a flexible structure (15), arranged so as to be applied on at least the part of the subject's body, including:
i. a distribution layer (13) comprising a first series of pouches (130) adjacent in pairs extending along one another, one of the pouches adjacent in pairs at least partially covering the other of the pouches adjacent in pairs;
ii. a generation layer (14), covering the distribution layer (13), comprising a second series of inflatable pouches (140) adjacent in pairs extending along one another; and,
- means (200) for generating a pulsation fluidially connected to the flexible structure in a leak-tight manner and arranged so as to create pulsation waves in the second series of pouches of the generation layer between the inner and outer layers,
**characterised in that** the first series of pouches (130) of the generation layer including pouches filled with an incompressible fluid enabling the propagation of the pulsation waves created within the generation layer by the means (200) for generating.

2. Device according to claim 1, **characterised in that** the pouches of the second series of pouches are mounted in a scaled structure.

3. Device according to claim 1 or 2, **characterised in that** each of the pouches of the second series of pouches (140) comprises an elastic flexible inner layer (102) on the side of the patient's body and a more rigid outer layer (104).

4. Device according to one of claims 1 to 3, **characterised in that** each of the inflatable pads includes a pulsation fluid intake duct (114) fluidically connected with means for generating a pulsation.

5. Device according to one of claims 1 to 4, **characterised in that** the flexible structure (15) includes a non-stretch flexible outer layer (11).

6. Device according to one of claims 1 to 5, **characterised in that** it includes a mask (500) arranged so as to be disposed on at least a part of a subject's face.

7. Device according to one of claims 1 à 5, **characterised in that** it includes pants (600).

8. Device according to one of claims 1 to 5, **characterised in that** it includes a jacket.

9. Device according to one of claims 1 to 5, **characterised in that** it includes a glove.

10. Device according to one of claims 1 to 5, **characterised in that** it includes a boot (606) or a stocking.

11. Non-invasive pulsatile circulatory support assembly covering several parts of a subject's body, **characterised in that** it comprises at least one device according to one of claims 1 to 10 for each of the parts of the subject's body.
